(19) Europäisches Patentamt / European Patent Office / Office européen des brevets

(11) **EP 3 591 543 B1**

(12) **FASCICULE DE BREVET EUROPEEN**

(45) Date de publication et mention
de la délivrance du brevet:
**06.09.2023 Bulletin 2023/36**

(21) Numéro de dépôt: **18181500.2**

(22) Date de dépôt: **03.07.2018**

(51) Classification Internationale des Brevets (IPC):
**G16C 20/10** (2019.01)      **G06F 17/13** (2006.01)

(52) Classification Coopérative des Brevets (CPC):
**G06F 17/13; G16C 20/10;** G16B 5/00

(54) **SYSTÈME ET PROCÉDÉ POUR LA SIMULATION D'UN PROCÉDÉ CHIMIQUE OU BIOCHIMIQUE**

SYSTEM UND VERFAHREN FÜR DIE SIMULATION EINES CHEMISCHEN ODER
BIOCHEMISCHEN VERFAHRENS

SYSTEM AND METHOD FOR SIMULATING A CHEMICAL OR BIOCHEMICAL PROCESS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(43) Date de publication de la demande:
**08.01.2020 Bulletin 2020/02**

(73) Titulaire: **YF1**
**54000 Nancy (FR)**

(72) Inventeurs:
• **NICOUD, Roger-Marc**
**54690 Lay-St-Christophe (FR)**
• **BERGER, Etienne**
**54000 Nancy (FR)**

(74) Mandataire: **August Debouzy**
**7, rue de Téhéran**
**75008 Paris (FR)**

(56) Documents cités:
**US-A1- 2005 216 248**

• **Tom Marlin: "Chapter 3: Mathematical Modelling
Principles", Process Control, 1999, pages 49-95,
XP055534861, Extrait de l'Internet:
URL:http://pc-textbook.mcmaster.ca/Marlin-
Ch03.pdf [extrait le 2018-12-14]**
• **S.-M. SUH ET AL: "Modeling particle formation
during low-pressure silane oxidation: Detailed
chemical kinetics and aerosol dynamics",
JOURNAL OF VACUUM SCIENCE AND
TECHNOLOGY: PART A., vol. 19, no. 3, mai 2001
(2001-05), pages 940-951, XP055534853,
MELVILLE, NY., US ISSN: 0734-2101, DOI:
10.1116/1.1355757**
• **ALDONA KRUPSKA: "Mathematical description
of the nonlinear chemical reactions with
oscillatory inflow to the reaction field", JOURNAL
OF CHEMICAL SCIENCES, vol. 127, no. 6, juin
2015 (2015-06), pages 1025-1034, XP055534884,
India ISSN: 0974-3626, DOI:
10.1007/s12039-015-0871-5**
• **Russell L. Herman: "A first course in differential
equations for scientists and engineers", , 25 juin
2017 (2017-06-25), pages 1-369, XP055534890,
Extrait de l'Internet:
URL:http://people.uncw.edu/hermanr/mat361/
ODEBook/ODE2017.pdf [extrait le 2018-12-14]**

**Description**

## DOMAINE TECHNIQUE

La présente invention concerne le domaine de la modélisation et de la simulation en génie des procédés. En particulier, la présente invention porte sur un système et un procédé de simulation par ordinateur d'un procédé chimique ou biochimique.

## ÉTAT DE LA TECHNIQUE

Les industriels de la chimie fine ou des biotechnologies doivent fréquemment estimer ou comparer des procédés de production impliquant plusieurs opérations et divers équipements à partir d'une quantité d'informations très limitée. C'est tout particulièrement le cas dans les premiers stades de développement. Notons que dans ces industries, lorsqu'un procédé de production doit être imaginé, il arrive fréquemment que les chances de mise sur le marché de la molécule visée soient très faibles, par exemple, pour des raisons d'échecs d'essais cliniques. Il existe donc de très nombreuses estimations technico-économiques à effectuer sur de nombreuses molécules qui ne seront produites qu'en faibles quantités et pendant une période de temps limitée.

Lorsque les industriels souhaitent obtenir simplement une simulation rapide d'un procédé chimique ou biochimique en l'absence de connaissances de l'état interne des équipements mettant en oeuvre le procédé, les managers et ingénieurs estiment les performances des différentes opérations à l'aide de connaissances empiriques ou même simplement d'intuition. Ces connaissances sont du type : la teneur en composé A en sortie du réacteur est de 50% sans que cette teneur soit reliée à une connaissance des conditions internes du réacteur ou bien le taux de récupération du composé A dans le séparateur est de 95% sans que cette teneur soit reliée à une connaissance des conditions internes du séparateur. Ces informations figées permettent de relier les quantités de sortie (aussi appelées quantités finales en régime transitoire ; par la suite on se référera indifféremment aux termes quantités finales ou quantités de sortie) aux quantités d'entrée (aussi appelées quantités initiales en régime transitoire ; par la suite on se référera indifféremment aux termes quantités initiales ou quantités d'entrée). Cette méthode ne fait pas appel à des bilans de matière ou de chaleur et ne prend donc pas en compte les états internes des équipements. Elle est donc incapable de prévoir une dynamique. A partir de la connaissance des quantités d'entrée du procédé il est possible de calculer de proche en proche, pour chaque opération, les quantités de sortie. On peut donc connaître à l'aide d'un simple calculateur, comme par exemple un tableur Excel, les quantités de sortie obtenues à l'issue du procédé.

Cette méthode présente l'avantage de la simplicité et de la rapidité de calcul. Cependant, elle présente plusieurs inconvénients :

- le nombre de scenarii envisageables est limité aux connaissances expérimentales des opérateurs, les optimisations et comparaisons fines entre les procédés ne sont donc pas possibles ;
- lorsque de nombreuses opérations se déroulent en série au cours du procédé, la simulation devient particulièrement complexe et imprécise. Cette complexité est accentuée en cas de boucle de recyclage de composés au cours du procédé ;
- les régimes transitoires ne sont pas pris en compte. Cette méthode est limitée aux systèmes continus ou aux systèmes discontinus par lots dans lesquels on ne prend pas en compte les régimes transitoires. Or bien souvent les quantités produites par les industriels de la chimie fine ou des biotechnologies ne permettent pas de recourir à des productions en continu (contrairement aux industries pétrochimiques par exemple).

Lorsque les industriels souhaitent connaître avec plus de précision les quantités finales et les performances d'un procédé il est nécessaire d'avoir recours à des méthodes éprouvées du génie des procédés, basées sur des modélisations détaillées, tels que Honeywell UNISIM®, Aspen HYSYS®. Ces modèles nécessitent la connaissance d'un très grand nombre de paramètres physico-chimiques en particulier thermodynamiques qui permettent de simuler le comportement et les performances du système en fonction des conditions internes. Ces modèles prennent en compte les bilans de matière et de chaleur et déterminent l'évolution de variables d'état interne aux équipements. Cette méthode bien que plus précise est très peu utilisée par les industriels de la chimie fine ou des biotechnologies car elle nécessite la prise en compte d'un grand nombre de variables d'état interne et le recours à des méthodes numériques très complexes pour un non- spécialiste. Le temps et les ressources nécessaires à la détermination des paramètres physico-chimiques et à la mise au point de l'outil de simulation sont trop importants pour un procédé qui pourrait par la suite n'être utilisé que pour des quantités très réduites.

Aucune des approches existantes à ce jour n'est satisfaisante. Elles sont soit limitées à des scenarii particulièrement simplistes, soit trop complexes et requièrent la détermination d'un trop grand nombre de paramètres. Il existe donc un besoin pour un système et un procédé pour la simulation par ordinateur d'un procédé chimique ou biochimique permettant de simuler des scénarii complexes, incluant plusieurs opérations, dans un temps raisonnable tout en limitant le nombre de paramètres physico-chimiques requis pour effectuer la simulation.

La présente invention s'inscrit dans ce cadre.

## RÉSUMÉ

Les modes de réalisation de l'invention offrent un outil industriel d'ingénierie des procédés de chimie fine. Une caractéristique technique fonctionnelle des modes de réalisation réside dans la simulation de procédés pour leur mise en oeuvre ultérieure dans des installations de production de produits chimiques ou biochimiques.

Les modes de réalisation permettent de prédire, de façon concrète le comportement de procédés chimiques ou biochimiques pour faire un certain nombre d'estimations, notamment sur leur faisabilité industrielle. Les modes de réalisation de l'invention permettent ainsi d'orienter le développement de procédés chimiques ou biochimiques avec une précision suffisante, et dans des temps et des coûts raisonnables, pour permettre d'estimer les chances de réussite de leur mise en oeuvre industrielle et ce, avant même la mise en place d'une installation et la mise en fonctionnement de cette installation.

Une mise en oeuvre purement intellectuelle d'une simulation de procédés chimiques ou biochimiques n'est pas possible. C'est en particulier le cas des simulations pour des besoins industriels. Il est en pratique impossible de réaliser les calculs nécessaires pour arriver à des résultats suffisamment précis et significatifs permettant de prendre des décisions industrielles comme par exemple la mise en place d'installations chimiques. En outre, la mise en oeuvre purement intellectuelle de la simulation prendrait un temps prohibitif, ne particulier pour caler les paramètres du modèle.

Or, sans une simulation assistée par ordinateur, il est impossible de tester de façon prédictive ou de faire un choix parmi une pluralité de projets de procédés chimiques ou biochimiques ceux qui offrent les meilleurs performances, et ce, dans un temps industriellement raisonnable.

Il n'existe aucune méthode purement intellectuelle, mathématique ou encore théorique qui permette de prédire de manière exhaustive et rapide le comportement d'un procédé de chimie fine.

Un but de l'invention est de permettre aux industries de la chimie fine et des biotechnologies l'utilisation de simulateurs pertinents en fournissant un outil utilisable par un non expert de la simulation, qui autorise la prise en compte d'un projet de production avec des connaissances de physico-chimie initialement quasi inexistantes.

Cet outil doit avoir la capacité d'aider l'utilisateur à identifier les points délicats ou limitants du procédé en évaluation afin de déterminer les connaissances à acquérir ou à utiliser afin d'atteindre un objectif donné, à affiner ces prévisions au fur à et mesure de la disposition des nouvelles connaissances. Cet outil devra de plus être fiable, facile à utiliser, et permettre une précision d'estimation des performances technico-économiques du procédé étudié qui soit adaptée au problème posé et qui pourra évoluer en fonction des besoins et de l'évolution du projet industriel.

A cette fin, selon un premier aspect, l'invention concerne un système selon la revendication 1.

L'invention permet d'obtenir une équation algébro-différentielle dont la solution converge vers une équation algébrique explicite de départ. Ainsi, il est possible d'utiliser des techniques de résolution d'équations algébro-différentielles pour obtenir des solutions d'équations algébrique. Il est alors possible d'utiliser les deux types de modélisations dans un même système de simulation et de bénéficier des avantages liés aux deux types d'équations en fonction du choix de l'utilisateur pour telle ou telle partie du modèle global du procédé chimique ou biochimique.

Le système selon l'invention permet de simuler des opérations représentées par l'intermédiaire d'équations algébriques explicites ou d'équations algébro-différentielles. Afin de résoudre un système combinant les deux types d'équations, le système selon l'invention génère une équation algébro-différentielle convergeant vers la solution de l'équation algébrique explicite de sorte à permettre la fusion des équations dans un unique système d'équations algébro-différentielles.

Selon un deuxième aspect, l'invention concerne un procédé selon la revendication 4.

Selon un troisième aspect, l'invention concerne un produit programme d'ordinateur comprenant des instructions qui, lorsque le programme est exécuté par un ordinateur, conduisent celui-ci à mettre en oeuvre les étapes du procédé selon le deuxième aspect de l'invention.

Selon un quatrième aspect, l'invention concerne un support de stockage de l'information non transitoire lisible par ordinateur comprenant des instructions qui lorsqu'elles sont exécutées par un ordinateur conduisent celui-ci à mettre en oeuvre les étapes du procédé selon le deuxième aspect de l'invention.

## DÉFINITIONS

Par « chimie fine » également appelée « chimie de spécialité » on entend dans le présent document une branche de l'industrie chimique qui synthétise des produits spécifiques, dans de faibles volumes de production, mais à haute valeur ajoutée, et répondant à des contraintes techniques, par exemple de pureté, élevées.

Par « biotechnologie » on entend des technologies de production de molécules par fermentation, cultures de cellules, extraction du milieu naturel.

Par « équation algébrique » on entend une équation ayant pour inconnue une ou plusieurs variables réelles.

Par « équation différentielle » on entend une équation ayant pour inconnue une ou plusieurs fonctions ; elle se présente sous la forme d'une relation entre ces fonctions inconnues et leurs dérivées successives.

Par « équation explicite » on entend une équation entre différentes variables où une variable est explicitée en fonction des autres.

Par « équation implicite » on entend une équation entre différentes variables où aucune variable n'est explicitée en fonction des autres.

Par « variable d'état interne », on entend une variable chimique qui décrit ce qui se passe à l'intérieur d'un équipement donné, par exemple la température, la pression, la concentration...

Par « variable d'état d'entrée » ou « variable d'état de sortie », on entend une variable chimique qui décrit ce qui rentre ou sort, respectivement, d'un équipement donné, par exemple la température, la pression, la concentration...

Par « pseudo-variable d'état interne » on entend une variable artificielle permettant de transformer des « équations algébriques explicites » simples reliant un vecteur de variables d'état de sortie à un vecteur de variables d'état d'entrée dans un formalisme mathématique identique à celui des modèles prédictifs utilisant des « équations algébro-différentielles ».

Par « modèle prédictif » on entend l'association d'un certain nombre d'équations algébriques et différentielles basées sur des concepts de chimie et ou de physique dont la résolution permet la détermination des variables d'état interne d'au moins une opération effectuée dans au moins un équipement.

Par « opération » on entend une transformation chimique ou biochimique permettant de passer d'un état d'entrée à un état de sortie (ou d'un état initial à un état final). Généralement un procédé chimique ou biochimique comprend une pluralité d'opérations.

Par "temps caractéristique », on entend le temps de mise en régime de l'opération qui pour les procédés chimique varie par exemple de quelques minutes à quelques heures.

## DESCRIPTION DES FIGURES

La figure 1 illustre un procédé chimique mettant en oeuvre plusieurs opérations O1, 02, 03, O4 et plusieurs équipements E1, E2, E3, E4.

La figure 2 illustre de façon schématique le système conformément à un mode de réalisation de l'invention.

La figure 3 est un diagramme illustrant des étapes pour effectuer la simulation conformément à un mode de réalisation de l'invention.

La figure 4 représente un diagramme bloc représentant un dispositif pour implémenter un ou plusieurs modes de réalisation de l'invention.

La figure 5 représente un schéma de réaction comprenant un réacteur avec une entrée In et deux sorties Out1, Out2.

## DESCRIPTION DÉTAILLÉE

Un procédé chimique peut être découpé en une pluralité d'opérations réalisées dans une pluralité d'équipements. Le nombre d'opérations peut être inférieur, supérieur ou égal au nombre d'équipements. En effet, le nombre d'opérations peut être supérieur au nombre d'équipements si plusieurs opérations sont effectuées dans le même équipement tandis que le nombre d'équipements peut être supérieur au nombre d'opérations si une opération nécessite plusieurs équipements.

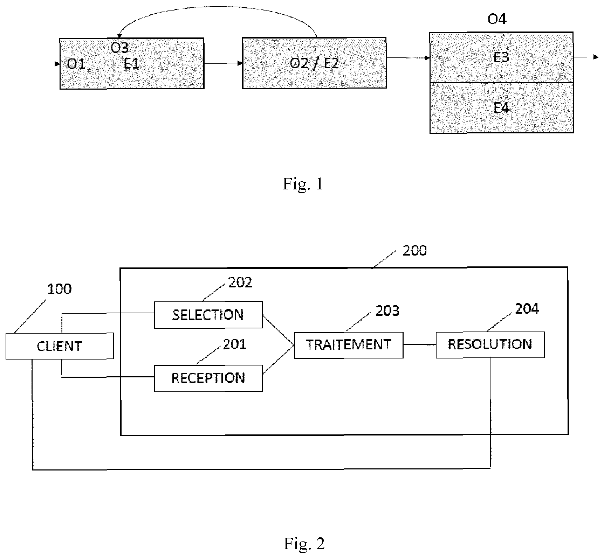

La **figure 1** illustre ces différents cas de figures. En raison de la boucle de retour, les opérations O1 et 03 sont réalisées au sein du même équipement E1 tandis que l'opération 04 est réalisée à l'aide de deux équipements E3 et E4 (à titre d'exemple non limitatif l'équipement E3 peut être un réacteur et l'équipement E4 un échangeur de chaleur).

Une opération (i) et un équipement (j) permettent de passer d'un état d'entrée ( $Y_e^{i/j}$ ) à un état de sortie ( $Y_s^{i/j}$ ). Les variables d'état d'entrée et d'état de sortie sont des vecteurs représentant des grandeurs chimiques en entrée et en sortie d'une opération et d'un équipement d'un procédé chimique ou biochimique.

Lorsqu'une simulation simple et rapide d'une opération et d'un équipement est souhaitée, l'opération et l'équipement sont décrits par des équations algébriques explicites reliant les états d'entrée ( $Y_e^{i/j}$ ) et de sortie ( $Y_s^{i/j}$ ) pour l'opération (i) et de l'équipement (j) : $Y_s^{i/j} = G_e(Y_e^{i/j})$ . Il n'y a aucune ambition prédictive dans cette approche.

Lorsqu'une simulation plus complète et précise est souhaitée, il est nécessaire de déterminer ce qui se passe à l'intérieur de l'équipement, et donc de déterminer un vecteur de variables d'état interne ($X^{i/j}$) pour une opération (i) et un équipement (j). Les variables d'état interne sont des vecteurs représentant des grandeurs chimiques internes d'une opération et d'un équipement d'un procédé chimique ou biochimique.

En chimie fine et/ou biotechnologie, les équipements fonctionnent généralement en régime transitoire, les variables d'état interne sont donc le plus souvent des solutions de systèmes d'équations algébriques et différentielles (système algébro-différentiel) qui représentent des bilans de matière, de chaleur ou des équilibres thermodynamiques par exemple.

Dans le cas général, l'opération et l'équipement sont décrits par un système d'équations différentielles faisant intervenir les variables d'état interne. Ces équations relient le vecteur de variables d'état interne ($X^{i/j}$) au vecteur de variables d'état d'entrée ( $Y_e^{i/j}$ ) pour l'opération (i) et l'équipement (j) :

$$P_{RT}^E \ (Y_e^{i/j}, \text{t}, X^{i/j}, \frac{dX^{i/j}}{dT})=0.$$

Le vecteur de variables d'état de sortie ( $Y_s^{i/j}$ ) qui peut dépendre du temps est alors déterminé explicitement à partir de la connaissance des variables d'état interne :

$$Y_s^{i/j}(t) = P_{RT}^E \ (\text{t}, X^{i/j})$$ dépendant lui-même du vecteur des variables d'état d'entrée

Notons qu'en régime permanent l'état final du vecteur des variables d'état de sortie s'obtient immédiatement par : $Y_s^{i/j}(t = \infty) = P_{RT}^E \ ( \ t = \infty, X^{i/j})$

où $t_\infty$ représente le temps de fin de l'opération dans un système discontinu ou un temps suffisamment grand dans un système continu.

Les équations décrivant les opérations et les équipements d'un procédé chimique ou biochimique doivent être résolues de façon couplée en prenant en compte les connexions entre toutes les opérations et tous les équipements au cours de l'ensemble du procédé. Dans le cas d'un système d'équations faisant intervenir les variables d'état interne, cette résolution peut être particulièrement longue en raison de problèmes de convergence mais surtout nécessite la connaissance de paramètres physico-chimiques dont la détermination peut-être longue et difficile.

Deux modes de simulation des opérations/équipements sont ainsi disponibles. L'un assez trivial repose sur de simples relations algébriques explicites entre variables d'état de sortie et d'entrée. L'autre beaucoup plus précis et prédictif impose la résolution d'équation algébro différentielles complexes et la connaissance de nombreux paramètres physico-chimiques. Il est donc souhaitable de pouvoir passer d'une approche détaillée à l'aide d'équations faisant intervenir les variables d'état interne à une approche simplifiée à l'aide d'équations algébriques explicites sans intervention des variables d'état interne pour différentes opérations au sein d'un même procédé. Il serait ainsi possible de gagner un temps précieux en choisissant de simuler au sein d'un procédé chimique ou biochimique une opération simple ou d'influence secondaire à l'aide d'une équation algébrique explicite et une opération plus complexe ou bien critique à l'aide d'un système algébro-différentiel faisant intervenir les variables d'état interne. Cependant, de par la structure mathématique des équations, il n'est pas possible d'intégrer ces deux types d'équations dans un même système de résolution général.

Pour permettre l'intégration de ces deux types d'équations dans un seul système de résolution, les inventeurs ont eu l'idée de remplacer les équations algébriques explicites par des équations algébro-différentielles faisant intervenir des pseudo-variables d'état interne. Les équations algébro-différentielles ainsi créées sont affectées d'une constante de temps très faible devant le temps caractéristique de l'opération. On notera que l'homme du métier connait le temps caractéristique de chaque opération du procédé et saura choisir une constante de temps faible par rapport à ce temps caractéristique (par exemple une constante de temps de l'ordre de la seconde). En choisissant une constante de temps faible, pour un temps bien supérieur à cette constante de temps, le terme différentiel s'annule en régime permanent et il ne reste à s'assurer que les autres termes de l'équation différentielle convergent vers la solution de l'équation algébrique explicite.

Grâce à cette transformation, les équations algébriques explicites peuvent être intégrées dans le système de simulation et l'utilisateur peut choisir entre une modélisation fine ou non de chaque opération. Ainsi, alors que passer d'un système algébrique à un système algébro-différentiel peut sembler être une complexification de la simulation, cela permet en fait d'apporter une souplesse dans la simulation sans pénaliser les performances de calcul. L'utilisateur peut opter pour l'une ou l'autre des modélisations, en utilisant un même module de résolution d'équations.

A titre d'illustration, l'équation algébrique explicite $Y_s^i = \alpha\, Y_e^i$ peut être remplacée par l'équation différentielle

$$\Theta\, \frac{d\tilde{X}^i}{dT} - \alpha Y_e^i + \tilde{X}^i = 0$$

, avec comme condition initiale $\tilde{X}^i(t=0) = 0$, où $\Theta$ est une constante de temps et $\tilde{X}^i$ une pseudo-variable d'état interne. Lorsque t est bien supérieur à la constante de temps, la solution $\tilde{X}^i(t)$ est constante, sa dérivée est donc nulle, et par conséquent $\alpha Y_e^i = \tilde{X}^i(t \gg \Theta)$, Pour un temps suffisamment long par rapport à la constante de temps, on retrouve donc la solution de l'équation algébrique explicite ; il suffit pour cela de poser : $Y_s^i = \tilde{X}^i(t \gg \Theta)$ .

L'opérateur peut donc choisir de décrire une opération à l'aide d'un système d'équations faisant intervenir les variables d'état interne ou bien une équation algébrique explicite. Si au moins une équation algébrique explicite est sélectionnée pour au moins une opération du procédé, le système selon l'invention remplace cette équation algébrique explicite en équation algébro-différentielle afin de pouvoir l'intégrer dans le système d'équation de l'ensemble du procédé.

La **figure 2** illustre un schéma-bloc du système selon un mode de réalisation de l'invention. Dans le contexte général de la mise en oeuvre du système, un dispositif client 100 se connecte au système de simulation 200 selon l'invention. Le système 200 selon l'invention comprend un module de réception 201, optionnellement un module de sélection 202, un module de traitement 203 et un module de résolution d'équations algébro-différentielles 204.

Le dispositif client 100 se connecte au module de réception 201 afin de communiquer au module de réception 201 une équation algébrique explicite représentant une opération chimique ou biochimique et reliant un vecteur de variables d'état d'entrée représentant des grandeurs chimiques initiales de ladite opération à un vecteur de variables d'état de sortie représentant des grandeurs chimiques finales de ladite opération.

Le module de réception 201 est connecté au module de traitement 203 afin de recevoir l'équation algébrique explicite représentant une opération chimique et biochimique et de créer une équation algébro-différentielle de sorte à ce que la solution en régime permanent de l'équation algébro-différentielle ainsi créée converge vers ledit vecteur de variables d'état de sortie selon l'équation algébrique explicite.

Le module de traitement 203 est connecté au module de résolution d'équation algébro-différentielle 204. Lorsque le module de traitement 203 a effectué la création de l'équation algébro-différentielle, le module de résolution 204 résout l'équation afin d'obtenir le vecteur de variables d'état de sortie de l'opération.

Le module de résolution d'équation algébro-différentielle 204 est connecté au dispositif client 100 afin de retourner le vecteur de variables d'état de sortie de l'opération.

Selon un mode de réalisation, le dispositif client 100 communique au module de réception 201 une équation algébrique explicite de la première opération et une équation algébro-différentielle de la première opération. Le dispositif client 100 est connecté au module de sélection 202 de sorte à sélectionner une simulation par l'intermédiaire de l'équation algébrique explicite ou une simulation par l'intermédiaire de l'équation algébro-différentielle. En fonction du mode sélectionné, le module de traitement créé une équation algébro-différentielle convergeant vers ledit vecteur de variables d'état de sortie selon l'équation algébrique explicite ou bien utilise l'équation algébro-différentielle existante.

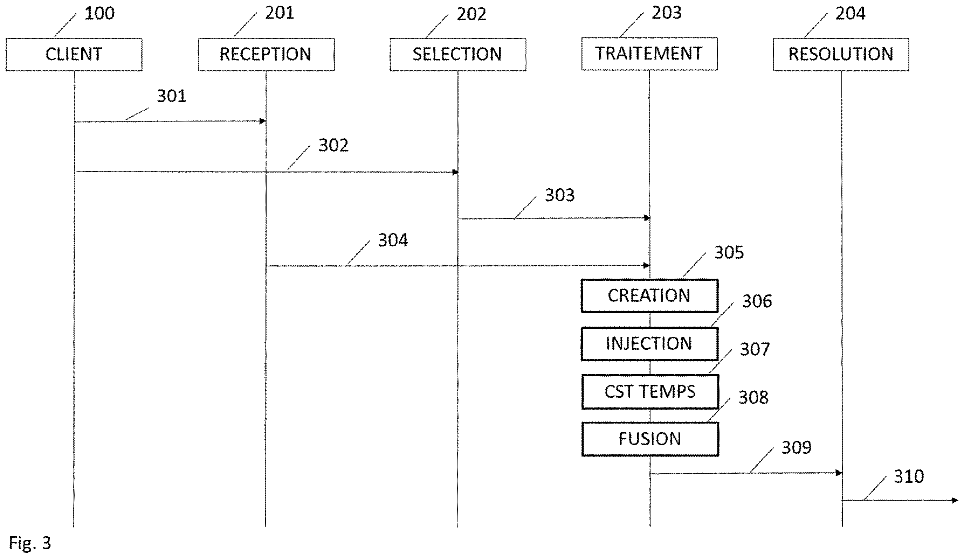

La **figure 3** illustre les étapes pour effectuer la simulation conformément à des modes de réalisation.

Le module client 100 communique avec le module de réception 201 à l'étape 301. Au cours de cette étape 301, le module client communique au module de réception une équation algébrique explicite représentant une première opération chimique ou biochimique et reliant un premier vecteur de variables d'état d'entrée représentant des grandeurs chimiques initiales de ladite première opération à un premier vecteur de variables d'état de sortie représentant des grandeurs chimiques finales de ladite première opération. Selon un mode de réalisation, au cours de cette étape 301, le dispositif client peut également communiquer une équation algébro-différentielle représentant ladite première opération chimique ou biochimique et reliant un premier vecteur de variables d'état d'entrée ; et un premier vecteur de variables d'état interne de ladite première opération en tant qu'inconnue de l'équation. Selon un mode de réalisation, au cours de cette étape 301, le dispositif client peut également communiquer une équation algébro-différentielle représentant une deuxième opération chimique ou biochimique et reliant un deuxième vecteur de variables d'état d'entrée ; et un deuxième vecteur de variables d'état interne de ladite première opération en tant qu'inconnue de l'équation.

Selon un mode de réalisation, le module client 100 communique avec le module de réception à l'étape 302. Lors de cette étape 302, le dispositif client sélectionne un mode de simulation. Le mode de simulation comprend soit la simulation de l'opération chimique par l'intermédiaire de l'équation algébro-différentielle soit par l'intermédiaire de l'équation algébrique explicite.

Lors de l'étape 303, le module de sélection 202 communique au module de traitement le mode de simulation sélectionné et le module de réception transmet 304 au module de traitement l'équation en fonction du mode de simulation sélectionné.

Si une simulation à partir d'une équation algébrique explicite est sélectionnée pour la première opération, le module de traitement effectue l'étape de création 305 d'une première équation algébro-différentielle reliant un premier vecteur de variables d'état d'entrée ; et un premier vecteur de variables d'état interne de ladite première opération en tant qu'inconnue de l'équation. Par la suite, le module de traitement injecte 306 dans la première équation algébro-différentielle l'expression du vecteur de variables d'état de sortie de ladite première opération selon ladite première équation algébrique explicite en tant que vecteur de pseudo-variables d'état interne de ladite première opération, la solution en régime permanent de ladite équation algébro-différentielle convergeant ainsi vers ledit premier vecteur de variables d'état de sortie selon la première équation algébrique explicite. Le module de traitement fixe ensuite à l'étape 307 la constante de temps. Cette constante de temps est fixée de façon à être très inférieure au temps caractéristique de la première opération. Le temps caractéristique d'une opération chimique ou biochimique varie typiquement de quelques minutes à quelques heures. Dans ces conditions, si la constante de temps est égale à une seconde, tout se passe comme si le système algébro-différentiel convergeait de façon instantanée vers la solution de l'équation algébrique explicite. Dans un mode de réalisation, la constante de temps est donnée par l'utilisateur via le module client 100 et le module de réception 201 au module de traitement 203 qui fixe alors la constante de temps de l'équation à la valeur indiquée par l'utilisateur.

Le module de traitement transmet ensuite l'équation algébro-différentielle ainsi obtenue au module de résolution pour la résolution de l'équation 309 afin d'obtenir le vecteur de variables d'état de sortie de la première opération.

Selon un mode de réalisation, le module de résolution transmet ce vecteur de variables d'état de sortie de la première opération au dispositif client au cours d'une étape 310.

Selon un mode de réalisation où le procédé comprend deux opérations dont une deuxième opération modélisée dès le départ par une équation algébro-différentielle, le module de traitement est configuré pour recevoir depuis le module de réception la deuxième équation algébro-différentielle au cours d'une étape 304 et pour fusionner la seconde équation algébro-différentielle avec la première équation algébro-différentielle au cours d'une étape 308. Le module de traitement transmet ensuite le système d'équations algébro-différentielles au module de résolution au cours d'une étape 309 qui va résoudre ce système et obtenir un vecteur de variables d'état de sortie du procédé comprenant les deux opérations.

Selon un mode de réalisation où une opération est représentée à la fois par une équation algébrique explicite et une équation algébro-différentielle, le dispositif client sélectionne à l'étape 302 un mode de simulation. Si la simulation par l'intermédiaire de l'équation algébrique explicite est sélectionnée, le module de traitement effectue les étapes de création 305, d'injection 306 et fixation de la constante de temps 307 puis l'étape de fusion 308 avec les équations algébro-différentielles des autres opérations du procédé. Si la simulation par l'intermédiaire de l'équation algébro-différentielle est sélectionnée, le module de traitement fusionne directement cette équation algébro-différentielle avec les équations algébro-différentielles des autres opérations du procédé. Ainsi, la présente invention permet de combiner une simulation précise à l'aide d'une équation algébro-différentielle pour certaines opérations critiques et la sélection d'une simulation moins précise à l'aide d'une équation algébrique explicite. La solution de cette équation algébrique explicite étant intégrée dans une équation algébro-différentielle afin de permettre la fusion des équations algébro-différentielles dans un unique système de résolution.

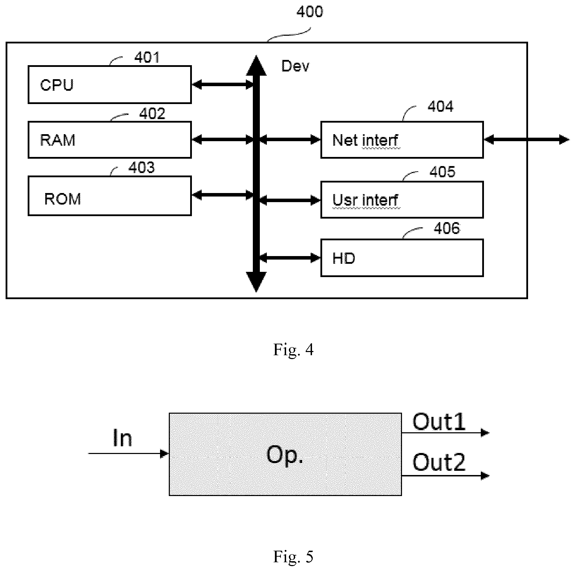

Les diverses parties du système décrites ci-dessus peuvent être mises en oeuvre par un ou plusieurs programmes d'ordinateur. Ainsi, chaque module peut correspondre à une routine d'un ou plusieurs programmes d'ordinateur. Le système est alors mis en oeuvre par un dispositif global 400 tel qu'illustré sur la **figure 4.**

Le dispositif 400 comprend un bus de communication relié à :

- une unité centrale de traitement 401 tel qu'un microprocesseur, autrement nommée CPU ;
- une mémoire à accès aléatoire 402, autrement nommée RAM, pour stocker un code exécutable du procédé des modes de réalisation de l'invention ainsi que les registres adaptés pour enregistrer les variables et les paramètres nécessaires à la mise en oeuvre du procédé conformément aux modes de réalisation, la capacité de la mémoire pouvant être augmentée par une RAM optionnelle connectée par exemple à un port d'expansion ;
- une mémoire à lecture seule 403, autrement nommée ROM, pour stocker les programmes d'ordinateur utilisés pour mettre en oeuvre les modes de réalisation de l'invention ;
- une interface de réseau 404, qui est typiquement connectée à un réseau de communication sur lequel des données numériques devant être traitées sont transmises ou reçues. L'interface de réseau 404 peut être une seule interface de réseau ou être composée d'un ensemble d'interfaces de réseau différentes (par exemple des interfaces filaires et sans-fil, ou différentes sortes d'interfaces filaires ou sans-fil). Les données sont écrites sur l'interface de réseau pour transmission ou lues à partir de l'interface réseau pour réception sous le contrôle de l'application logicielle fonctionnant dans le CPU 401 ;
- une interface utilisateur 405 pour la réception des saisies d'un utilisateur ou pour l'affichage d'information à l'utilisateur ;

- un disque dur 406 autrement nommé HD
- un module entrée/sortie 407 (autrement nommé I/O) pour envoyer/recevoir des données de/vers des dispositifs tels qu'une source vidéo ou un écran d'affichage.

Le code exécutable peut être stocké, soit dans la mémoire à lecture seule 403, soit sur le disque dur 406, ou sur un support numérique amovible tel qu'un disque par exemple. Selon une variante, le code exécutable des programmes peut être reçu au moyen d'un réseau de communication, via l'interface de réseau 404, afin d'être stocké sur un des supports de stockage du dispositif de communication 400, tel que le disque dur 406, avant d'être exécuté.

L'unité centrale de traitement 401 est adaptée pour contrôler et diriger l'exécution des instructions ou des portions de code logiciel du programme conformément aux modes de réalisation de l'invention, lesquelles instructions sont stockées sur l'un des supports de stockage précités. Après avoir été mise en service, la CPU 401 est capable d'exécuter les instructions à partir de la mémoire RAM principale 402 en rapport avec une application logicielle par exemple après que ces instructions ont été chargées à partir du programme ROM 403 ou sur le disque dur (HD) 406. Cette application logicielle, lorsqu'elle est exécutée par la CPU 401, amène les étapes de procédé à être mises en oeuvre conformément aux modes de réalisation.

## EXEMPLES

Dans cet exemple on considère une opération de réaction (Op.) avec séparation des produits en aval comme illustré sur la **figure 5.** La réaction est la suivante : $S1 + S3 \rightarrow S2 + S4$ avec consommation totale de S3. Les quantités d'entrée du réacteur sont données par le suivant :

| Espèce | Quantités entrantes (ou initiales) (In) en moles |
|--------|--------------------------------------------------|
| S1     | 10                                               |
| S2     | 0                                                |
| S3     | 5                                                |
| S4     | 0                                                |

Si la réaction est supposée totale, alors après la réaction, les quantités des différentes espèces sont données par :

| Espèce | Quantités post réaction en moles |
|--------|----------------------------------|
| S1     | 5                                |
| S2     | 5                                |
| S3     | 0                                |
| S4     | 5                                |

En aval de la réaction les espèces sont réparties entre deux sorties, nommées Out1 et Out 2, selon les ratios de répartition donnés dans le suivant :

| Espèce | Ratio de répartition dans le premier flux de sortie (Out1) | Ratio de répartition dans le second flux de sortie (Out2) |
|--------|------------------------------------------------------------|-----------------------------------------------------------|
| S1     | 10%                                                        | 90%                                                       |
| S2     | 5%                                                         | 95%                                                       |
| S3     | 50%                                                        | 50%                                                       |
| S4     | 95%                                                        | 5%                                                        |

Les ratios de répartition d'une espèce donnent les fractions de cette espèce contenue dans les différents flux de sortie. Pour chaque espèce, la somme des ratios de répartition égale 100 %.

Les vecteurs de variables d'état d'entrée, de première sortie et de seconde sortie contiennent les nombres de moles des différentes espèces et sont respectivement :

$$Y_E = \begin{pmatrix} m_{S1}^{In} \\ m_{S2}^{In} \\ m_{S3}^{In} \\ m_{S4}^{In} \end{pmatrix}, Y_{S1} = \begin{pmatrix} m_{S1}^{Out1} \\ m_{S2}^{Out1} \\ m_{S3}^{Out1} \\ m_{S4}^{Out1} \end{pmatrix}, Y_{S2} = \begin{pmatrix} m_{S1}^{Out2} \\ m_{S2}^{Out2} \\ m_{S3}^{Out2} \\ m_{S4}^{Out2} \end{pmatrix}$$

On peut déterminer les différents vecteurs de variables d'état :

$$Y_E = \begin{pmatrix} 10 \\ 0 \\ 5 \\ 0 \end{pmatrix}$$

A l'entrée (état initial) du système

$$Y_{Après\ react} = \begin{pmatrix} 5 \\ 5 \\ 0 \\ 5 \end{pmatrix}$$

Après la réaction (avant séparation) :
A la sortie (état final) en appliquant les ratios de séparation au mélange post réactionnel :

$$Y_{S1} = \begin{pmatrix} 0.5 \\ 0.25 \\ 0 \\ 4.75 \end{pmatrix}, Y_{S2} = \begin{pmatrix} 4.5 \\ 4.75 \\ 0 \\ 0.25 \end{pmatrix}$$

On obtient ainsi les équations algébriques explicites (Ex1, Ex2) suivantes :

$$(Ex1)\ A_{S1} = \begin{pmatrix} 0,1 & 0 & -0,1 & 0 \\ 0 & 0,05 & 0,05 & 0 \\ 0 & 0 & 0 & 0 \\ 0 & 0 & 0,95 & 0,95 \end{pmatrix};\ Y_{S1} = A_{S1}Y_E$$

$$(Ex2)\ A_{S2} = \begin{pmatrix} 0,9 & 0 & -0,9 & 0 \\ 0 & 0,95 & 0,95 & 0 \\ 0 & 0 & 0 & 0 \\ 0 & 0 & 0,05 & 0,05 \end{pmatrix};\ Y_{S2} = A_{S2}Y_E$$

Dans cet exemple, on considère que lors de la séparation les espèces sont acheminées avant leur sortie vers deux cellules parfaitement homogènes dont les nombres de moles des différentes espèces sont regroupés dans les vecteurs $\tilde{X}_1$ et $\tilde{X}_2$. On considère également que la matière soutirée des cellules par les sorties (Out1 et Out2) est de la même composition que celle des cellules. On peut alors écrire :

$$Y_{S1} = \tilde{X}_1 \text{ et } Y_{S2} = \tilde{X}_2.$$

On peut transformer le système (Ex1) (Ex2) en système différentiel en introduisant des pseudo-variables d'état interne ($\tilde{X}_1$ et $\tilde{X}_2$) et en posant:

$$(Ex3)\ -\theta \frac{d\tilde{X}_1}{dt} - \tilde{X}_1 + A_{S1}Y_E = 0$$

et

$$(\text{Ex4}) \; -\theta\frac{d\widetilde{X}_2}{dt} - \widetilde{X}_2 + A_{S2}Y_E = 0 \; ;$$

où $\theta$ est la constante de temps arbitraire des cellules homogènes ; lorsque t devient très supérieur à $\theta$ le terme différentiel s'annule et les pseudo-variables d'état convergent vers:

$$\widetilde{X}_1 = A_{S1}Y_E \text{ et } \widetilde{X}_2 = A_{S2}Y_E$$

Pour retrouver (Ex1) et (Ex2) il suffit de poser :

$$Y_{S1} = \widetilde{X}_1 \text{ et } Y_{S2} = \widetilde{X}_2.$$

En choisissant une constante de temps faible, on passe ainsi des équations algébriques explicites (Ex1 et Ex2) à des équations différentielles (Ex3 et Ex4). Ces équations différentielles, faisant intervenir des pseudo-variables d'état interne, peuvent ensuite être introduites dans le système d'équations différentielles simulant l'intégralité du procédé chimique ou biochimique.

**Revendications**

1. Système (200) pour la simulation par ordinateur d'un procédé chimique ou biochimique pour leur mise en oeuvre ultérieure dans des installations de production de produits chimiques ou biochimiques, ledit procédé comprenant au moins une première opération chimique ou biochimique et une deuxième opération chimique ou biochimique ; ledit système comprenant :

   - un module de résolution (204) configuré pour la résolution d'équations algébro-différentielles,
   - un module de réception (201) configuré pour recevoir une première équation algébrique explicite représentant ladite première opération chimique ou biochimique et reliant un premier vecteur de variables d'état d'entrée représentant des grandeurs chimiques initiales de ladite première opération à un premier vecteur de variables d'état de sortie représentant des grandeurs chimiques finales de ladite première opération ; et
   - un module de traitement (203) configuré pour :

      o dans une première équation algébro-différentielle reliant :

         ▪ ledit premier vecteur de variables d'état d'entrée ; et
         ▪ un premier vecteur de variables d'état interne de ladite première opération en tant qu'inconnue de l'équation ;

      o injecter dans ladite première équation algébro-différentielle l'expression du vecteur de variables d'état de sortie de ladite première opération selon ladite première équation algébrique explicite en tant que vecteur de pseudo-variables d'état interne de ladite première opération, la solution en régime permanent de ladite équation algébro-différentielle ainsi obtenue convergeant ainsi vers ledit premier vecteur de variables d'état de sortie selon la première équation algébrique explicite,
      o fixer une constante de temps de ladite équation algébro-différentielle ainsi obtenue comme étant inférieure à un temps caractéristique de ladite première opération, et
      o mettre en oeuvre le module de résolution sur l'équation algébro-différentielle ainsi obtenue pour calculer ledit premier vecteur de variables d'état de sortie ;

   dans lequel :

   - ledit module de réception (201) est en outre configuré pour recevoir une deuxième équation algébro-différentielle représentant ladite deuxième opération chimique ou biochimique reliant :

o un deuxième vecteur de variables d'état d'entrée représentant des grandeurs chimiques initiales de ladite deuxième opération ; et
o un deuxième vecteur de variables d'état interne de ladite deuxième opération en tant qu'inconnue de l'équation ;

la solution en régime permanent de ladite deuxième équation algébro-différentielle convergeant vers un deuxième vecteur de variables d'état de sortie représentant des grandeurs chimiques finale de ladite deuxième opération,
- ledit module de traitement (203) est en outre configuré

o pour fusionner lesdites première et deuxième équations algébro-différentielles et
o pour mettre en oeuvre le module de résolution (204) sur la fusion desdites première et deuxième équations algébro-différentielles ainsi obtenues pour calculer un vecteur de variables d'état de sortie du procédé.

2. Système (200) selon la revendication 1 comprenant en outre un module de sélection (202) configuré pour sélectionner un mode de simulation et dans lequel le module de traitement (203) est configuré pour réaliser ladite injection en fonction d'un mode sélectionné.

3. Procédé de simulation par ordinateur d'un procédé chimique ou biochimique
pour leur mise en oeuvre ultérieure dans des installations de production de produits chimiques ou biochimiques, ledit procédé comprenant au moins une première opération chimique ou biochimique et une deuxième opération chimique ou biochimique ; ledit procédé comprenant les étapes suivantes :

- la réception (301) d'une première équation algébrique explicite représentant ladite première opération chimique ou biochimique et reliant un premier vecteur de variables d'état d'entrée représentant des grandeurs chimiques initiales de ladite première opération à un premier vecteur de variables d'état de sortie représentant des grandeurs chimiques finales de ladite première opération ;
- la création (305) d'une première équation algébro-différentielle reliant :

o ledit premier vecteur de variables d'état d'entrée ; et
o un premier vecteur de variables d'état interne de ladite première opération en tant qu'inconnue de l'équation ;

- l'injection (306) dans ladite première équation algébro-différentielle de l'expression du vecteur de variables d'état de sortie de ladite première opération selon ladite première équation algébrique explicite en tant que vecteur de pseudo-variables d'état interne de ladite première opération, la solution en régime permanent de ladite équation algébro-différentielle ainsi obtenue convergeant vers ledit premier vecteur de variables d'état de sortie selon la première équation algébrique explicite,
- la fixation (307) d'une constante de temps de ladite équation algébro-différentielle ainsi obtenue inférieure à un temps caractéristique de ladite première opération ;
- la résolution (309) de l'équation algébro-différentielle ainsi obtenue pour calculer ledit premier vecteur de variables d'état de sortie ;

ledit procédé comprenant en outre les étapes suivantes :

- la réception (301) d'une deuxième équation algébro-différentielle représentant ladite deuxième opération chimique ou biochimique reliant

o un deuxième vecteur de variables d'état d'entrée représentant des grandeurs chimiques initiales de ladite deuxième opération ; et
o un deuxième vecteur de variables d'état interne de ladite deuxième opération en tant qu'inconnue de l'équation ;

la solution en régime permanent de ladite deuxième équation algébro-différentielle convergeant vers un deuxième vecteur de variables d'état de sortie représentant des grandeurs chimiques finale de ladite deuxième opération ;
- la fusion (308) des première et deuxième équations algébro-différentielles ;
- la résolution (309) de la fusion desdites première et deuxième équations algébro-différentielles ainsi obtenues pour calculer un vecteur de variables d'état de sortie du procédé.

4. Procédé de simulation selon la revendication 3, comprenant en outre une étape de sélection (302) d'un mode de simulation, les étapes de création, d'injection et de fixation d'une constante de temps (305, 306, 307) étant réalisées en fonction du mode sélectionné.

5. Produit programme d'ordinateur comprenant des instructions qui, lorsque le programme est exécuté par un ordinateur, conduisent celui-ci à mettre en oeuvre les étapes du procédé selon l'une quelconque des revendications 3 ou 4.

6. Support de stockage de l'information non transitoire lisible par ordinateur comprenant des instructions qui lorsqu'elles sont exécutées par un ordinateur conduisent celui-ci à mettre en oeuvre les étapes du procédé selon la revendication 3 ou 4.

**Patentansprüche**

1. System (200) zur rechnergestützten Simulation eines chemischen oder biochemischen Verfahrens zur späteren Ausführung in Erzeugungseinrichtungen für chemische bzw. biochemische Produkte, wobei das Verfahren mindestens einen ersten chemischen bzw. biochemischen Vorgang und einen zweiten chemischen bzw. biochemischen Vorgang umfasst; wobei das System umfasst:

- ein Lösungsmodul (204), das zum Lösen algebraischer Differentialgleichungen konfiguriert ist,
- Ein Empfangsmodul (201), das derart konfiguriert ist, dass es eine den ersten chemischen bzw. biochemischen Vorgang darstellende erste ausdrückliche algebraische Gleichung, die einen chemische Ausgangsgrößen des ersten Vorgangs darstellende Vektor für Ausgangszustandsvariablen mit einem die letztendlichen chemischen Größen des ersten Vorgangs darstellenden ersten Endzustandsvariablenvektor verbindet, empfängt, und
- ein Bearbeitungsmodul (203), das derart konfiguriert ist, dass es

∘ in einer ersten algebraischen Differentialgleichung zur Verbindung

▪ des ersten Ausgangszustandsvariablenvektors mit
▪ einem ersten Vektor von Variablen, die den internen Zustand des ersten Vorgangs betreffen, als unbekannter Größe,

∘ in die erste algebraische Differentialgleichung den Ausdruck des Endzustandsvariablenvektors des ersten Vorgangs gemäß der ersten ausdrücklichen algebraischen Gleichung als Vektor von Pseudovariablen des internen Zustands des ersten Vorgangs einführt, wobei die derart ermittelte Lösung der algebraischen Differentialgleichung im stationären Zustand in Richtung des ersten Endzustandsvariablenvektors gemäß der ersten ausdrücklichen algebraischen Gleichung konvergiert,
∘ eine Zeitkonstante der derart ermittelte algebraischen Differentialgleichung unterhalb einer charakteristischen Zeit des ersten Vorgangs festsetzt und
∘ das Lösungsmodul die derart ermittelte algebraische Differentialgleichung bearbeiten lässt, um den ersten Endzustandvariablenvektor zu errechnen, wobei

- das Empfangsmodul (201) ferner derart konfiguriert ist, dass es eine zweite algebraische Differentialgleichung empfängt, die den zweiten chemischen bzw. biochemischen Vorgang darstellt, welche

∘ einen zweiten Ausgangszustandsvariablenvektor, der chemische Ausgangsgrößen des zweiten Vorgangs darstellt mit
∘ einem ersten Vektor von Variablen, die den internen Zustand des ersten Vorgangs betreffen, als unbekannter Größe, verbindet,

wobei die Lösung der zweiten algebraischen Differentialgleichung im stationären Zustand in Richtung eines zweiten Endzustandsvariablenvektor, der chemische Endgrößen des zweiten Vorgangs darstellt, konvergiert,
- wobei das Bearbeitungsmodul (203) ferner konfiguriert ist zum

∘ Fusionieren der ersten und zweiten algebraischen Differentialgleichungen und
∘ Ausführen des Lösungsmoduls (204) an der Fusion der derart ermittelten ersten und zweiten algebraischen Differentialgleichung, um einen Ausgangs- und Endvariablenvektor des Vorgangs zu errechnen.

2. System (200) nach Anspruch 1, ferner umfassend ein Auswahlmodul (202), das derart konfiguriert ist, dass es einen Simulationsmodus auswählt, wobei das Bearbeitungsmodul (203) zur Ausführung der Einführung als Funktion eines ausgewählten Moduls konfiguriert ist.

3. Verfahren zur rechnergestützten Simulation eines chemischen oder biochemischen Verfahrens zur späteren Ausführung in Erzeugungseinrichtungen für chemische bzw. biochemische Produkte, wobei das Verfahren mindestens einen ersten chemischen bzw. biochemischen Vorgang und einen zweiten chemischen bzw. biochemischen Vorgang umfasst; wobei das Verfahren folgende Schritte umfasst:

   - Empfangen (301) einer den ersten chemischen bzw. biochemischen Vorgang darstellenden ersten ausdrücklichen algebraischen Gleichung, die einen chemische Ausgangsgrößen des ersten Vorgangs darstellende Vektor für Ausgangszustandsvariablen mit einem die letztendlichen chemischen Größen des ersten Vorgangs darstellenden ersten Endzustandsvariablenvektor verbindet,
   - Erstellen (305) einer ersten algebraischen Differentialgleichung zur Verbindung:

      ◦ des ersten Ausgangszustandsvariablenvektors mit
      ◦ einem ersten Vektor von Variablen, die den internen Zustand des ersten Vorgangs betreffen, als unbekannter Größe,

   - Einführen (306) des Ausdrucks des Endzustandsvariablenvektors des ersten Vorgangs gemäß der ersten ausdrücklichen algebraischen Gleichung als Vektor von Pseudovariablen des internen Zustands des ersten Vorgangs in die erste algebraische Differentialgleichung, wobei die derart ermittelte Lösung der algebraischen Differentialgleichung im stationären Zustand in Richtung des ersten Endzustandsvariablenvektors gemäß der ersten ausdrücklichen algebraischen Gleichung konvergiert,
   - Festsetzen (307) einer Zeitkonstante der derart ermittelte algebraischen Differentialgleichung unterhalb einer charakteristischen Zeit des ersten Vorgangs;
   - Auflösen (309) der derart ermittelten algebraischen Differentialgleichung, um den ersten Endzustandsvariablenvektor zu errechnen;

   wobei das Verfahren ferner folgende Schritte umfasst:

   - Empfangen (301) einer zweiten algebraischen Differentialgleichung, die den zweiten chemischen bzw. biochemischen Vorgang darstellt, zur Verbindung:

      ◦ eines zweiten Ausgangszustandsvariablenvektors, der chemische Ausgangsgrößen des zweiten Vorgangs darstellt, mit
      ◦ einem ersten Vektor von Variablen, die den internen Zustand des ersten Vorgangs betreffen, als unbekannter Größe,

   wobei die Lösung der zweiten algebraischen Differentialgleichung im stationären Zustand in Richtung eines zweiten Endzustandsvariablenvektor, der chemische Endgrößen des zweiten Vorgangs darstellt, konvergiert,
   - Fusionieren (308) der ersten und zweiten algebraischen Differentialgleichung;
   - Auflösen (309) der derart errechneten Fusion der ersten und zweiten algebraischen Differentialgleichungen, um einen Vektor für Endzustandsvariablen des Verfahrens zu ermitteln.

4. Verfahren nach Anspruch 3, ferner umfassend Auswählen (302) eines Simulationsmodus, wobei die Schritte des Erstellens, Einführens und Festsetzens einer Zeitkonstante (305, 306, 307) in Abhängigkeit des ausgewählten Modus ausgeführt werden.

5. Computerprogrammprodukt, umfassend Befehle, die bei Ausführung des Programms durch einen Computer diesen dazu veranlassen, die Schritte des Verfahrens nach einem der Ansprüche 3 oder 4 auszuführen.

6. Nicht flüchtiger, computerlesbarer Datenträger, umfassend Befehle, die bei Ausführung durch einen Computer diesen dazu veranlassen, die Schritte des Verfahrens nach Anspruch 3 oder 4 auszuführen.

**Claims**

1. A system (200) for the computer simulation of a chemical or biochemical process for the subsequent implementation thereof in facilities for producing chemical or biochemical products, said process comprising at least a first chemical or biochemical operation and a second chemical or biochemical operation; said system comprising:

   - a solver module (204) configured to solve differential-algebraic equations,
   - a reception module (201) configured to receive a first explicit algebraic equation representing said first chemical or biochemical operation and relating a first vector of input state variables representing initial chemical amounts of said first operation to a first vector of output state variables representing final chemical amounts of said first operation; and
   - a processing module (203) configured to:

     ○ in a first differential-algebraic equation relating:

       ▪ said first vector of input state variables; and
       ▪ a first vector of internal state variables of said first operation as an unknown of the equation;

     ○ inject, into said first differential-algebraic equation, the expression of the vector of output state variables of said first operation according to said first explicit algebraic equation as a vector of internal state pseudo-variables of said first operation, the steady-state solution of said differential-algebraic equation thus obtained thus converging toward said first vector of output state variables according to the first explicit algebraic equation,
     ○ set a time constant of said differential-algebraic equation thus obtained as being lower than a characteristic time of said first operation, and
     ○ implement the solver module on the differential-algebraic equation thus obtained to compute said first vector of output state variables,

   wherein:

   - said reception module (201) is further configured to receive a second differential-algebraic equation representing said second chemical or biochemical operation relating:

     ○ a second vector of input state variables representing initial chemical amounts of said second operation; and
     ○ a second vector of internal state variables of said second operation as an unknown of the equation;

   the steady-state solution of said second differential-algebraic equation converging toward a second vector of output state variables representing final chemical amounts of said second operation,
   - said processing module (203) is further configured

     ○ to merge said first and second differential-algebraic equations, and
     ○ to implement the solver module (204) on the merger of said first and second differential-algebraic equations thus obtained to compute a vector of output state variables of the process.

2. The system (200) according to claim 1, further comprising a selection module (202) configured to select a simulation mode and wherein the processing module (203) is configured to carry out said injection according to a selected mode.

3. A method for computer simulation of a chemical or biochemical process for the subsequent implementation thereof in facilities for producing chemical or biochemical products, said process comprising at least a first chemical or biochemical operation and a second chemical or biochemical operation; said method comprising the following steps:

   - receiving (301) a first explicit algebraic equation representing said first chemical or biochemical operation and relating a first vector of input state variables representing initial chemical amounts of said first operation to a first vector of output state variables representing final chemical amounts of said first operation; and
   - creating (305) a first differential-algebraic equation relating:

     ○ said first vector of input state variables; and

◦ a first vector of internal state variables of said first operation as unknown of the equation;

- injecting (306), into said first differential-algebraic equation, the expression of the vector of output state variables of said first operation according to said first explicit algebraic equation as a vector of internal state pseudo-variables of said first operation, the steady-state solution of said differential-algebraic equation thus obtained converging toward said first vector of output state variables according to the first explicit algebraic equation,
- setting (307) a time constant of said differential-algebraic equation thus obtained as being lower than a characteristic time of said first operation;
- solving (309) the differential-algebraic equation thus obtained to compute said first vector of output state variables;

said method further comprising the following steps:

- receiving (301) a second differential-algebraic equation representing said second chemical or biochemical operation relating

◦ a second vector of input state variables representing initial chemical amounts of said second operation; and
◦ a second vector of internal state variables of said second operation as an unknown of the equation;

the steady-state solution of said second differential-algebraic equation converging toward a second vector of output state variables representing final chemical amounts of said second operation;
- merging (308) said first and second differential-algebraic equations;
- solving (309) the merger of said first and second differential-algebraic equations thus obtained to compute a vector of output state variables of the method.

4. The simulation method according to claim 3, further comprising a step for selecting (302) a simulation mode, the steps of creation, injection and setting a time constant (305, 306, 307) being carried out according to the selected mode.

5. A computer program product comprising instructions that, when the program is run by a computer, lead the latter to implement the steps of the method according to any one of claims 3 or 4.

6. A medium for storing computer-readable non-transient information comprising instructions that, when run by a computer, lead the latter to implement the steps of the method according to claim 3 or 4.

O4

O3
O1　　　E1

O2 / E2

E3

E4

Fig. 1

200

100

202

SELECTION

203

204

CLIENT

201

TRAITEMENT

RESOLUTION

RECEPTION

Fig. 2

Fig. 3

400

401

CPU

402

RAM

403

ROM

Dev

404

Net interf

405

Usr interf

406

HD

Fig. 4

In → Op. → Out1
          → Out2

Fig. 5